# EUROPEAN PATENT SPECIFICATION

(11) **EP 2 387 399 B1**
(45) Date of publication and mention of the grant of the patent: **30.04.2014**
(21) Application number: 09795450.7
(22) Date of filing: 23.12.2009
(51) Int. Cl.: A61K 31/385, A61K 38/44, A61P 25/02

(54) **FOOD SUPPLEMENT FOR THE TREATMENT OF NEUROPATHIES**
NAHRUNGSERGÄNZUNGSMITTEL ZUR BEHANDLUNG VON NEUROPATHIEN
COMPLÉMENT ALIMENTAIRE DESTINÉ AU TRAITEMENT DE NEUROPATHIES

(30) Priority: 13.01.2009 IT MI20090023
(43) Date of publication of application: 23.11.2011
(73) Proprietor: Giellepi S.p.A., 20123 Milano (IT)
(72) Inventor: TERRUZZI, Carlo, I-20040 Briosco (IT)
(74) Representative: Montelatici, Linda Anna
(86) International application number: PCT/EP2009/067881
(87) International publication number: WO 2010/081622

(56) References cited:
- WO-A-94/06428
- WO-A-2005/115379
- WO-A2-03/039452
- ZIEGLER DAN: "Thioctic acid for patients with symptomatic diabetic polyneuropathy: a critical review" TREATMENTS IN ENDOCRINOLOGY, ADIS INTERNATIONAL, AUCKLAND, NZ, [Online] vol. 3, no. 3, 1 June 2004 (2004-06-01), pages 173-189, XP008105512 ISSN: 1175-6349 Retrieved from the Internet: URL:http://endocrinology.adisonline.com/pt /re/end/abstract.00024677-20040 3030-00005.htm>
- EROPKIN M YU ET AL: "Cytoprotective action of antihypoxic and antioxidant preparations in human cultured cells under conditions of provoked cytotoxic response" VOPROSY MEDITSINSKOI KHIMII, vol. 45, no. 5, September 1999 (1999-09), pages 384-388, XP008106267 ISSN: 0042-8809
- KUCHMEROVSKYY M ET AL: "Alpha-lipoic acid provides neuroprotection from diabetes-related oxidatuve injury of sciatic nerve" DIABETOLOGIA, vol. 45, no. Supplement 2, August 2002 (2002-08), page A 325, XP008106265 & 38TH ANNUAL MEETING OF THE EUROPEAN ASSOCIATION FOR THE STUDY OF DIABETES (EASD); BUDAPEST, HUNGARY; SEPTEMBER 01-05, 2002 ISSN: 0012-186X
- TANKOVA TSVETALINA ET AL: "Alpha-lipoic acid in the treatment of autonomic diabetic neuropathy (controlled, randomized, open-label study)." ROMANIAN JOURNAL OF INTERNAL MEDICINE = REVUE ROUMAINE DE MÉDECINE INTERNE 2004, vol. 42, no. 2, 2004, pages 457-464, XP008106411 ISSN: 1220-4749

## Description

The present invention relates to a composition of substances which is particularly useful for administration to subjects suffering from neuropathies.

Compositions useful for administration to subjects suffering from particular deficiency conditions or in a state of increased need of specific nutrients are generally known.

In the case of metabolic diseases such as diabetes, a lack of fundamental substances for the metabolism can occur and result in a need of specific nutrients. Poly-neuropathies are, among other diseases, very common among subjects suffering from diabetes. These can also be caused by a nutritional deficit and arc defined acquired neuropathies, or by inherited diseases and are therefore defined inherited neuropathies, and also in these cases a need of specific nutrients can result.

In particular, genetic diseases are at the base of inheritary neuropathies, whereas the acquired neuropathies are due to diseases acquired in the course of life, or caused by a nutritional deficit. The common denominator of acquired neuropathies is that the symptoms are bound to a severe and/or protracted oxidative stress, independently on the affected structures. It is known that oxidative stress is associated to an increase of the free radicals, that are the toxic oxygen species which, by seriously damaging the host cells, arc cause or concomitant cause of a number of pathologies, including those due to ageing of the subject.

It is also known that the toxic effects of the free radicals can be canceled by antioxidant substances, or prevented by substances which prevent their formation. The administration of substances which cancel the damaging effects of the free radicals or prevent their formation can be beneficial for treating people suffering from neuropathies whose origin is often referable to the same free radicals.

Many substances are known in nature which have an antioxidant effect, and some of these substances are also used for the preparation of medicines or food supplements or even dermo-cosmetic products.

α-Lipoic acid and polyphenols of vegetal origin, vitamins, enzymes such as superoxidodismutase, in the following indicated with the acronym SOD, can be mentioned among substances having an antioxidant effect.

The selection of such substances having an antioxidant effect seems often to be more motivated by the quantity of the antioxidating substances than by their particular effect, thus resulting in compositions comprising many substances being very different from each other. Consequently, in order to be effective, these compositions have to be administered in high quantity of doses per day in order to avoid too bulky dosage unities.

It is also known document US2003/0228299 describing a composition comprising racemic α-lipoic acid and SOD for the treatment and /or prevention of diseases of the eye surface. the weight ratio between the quantity of SOD and the quantity of α-lipoic acid of the composition according to US2003/0228299 is comprised in the range of 10000 and 0,005.

The need to provide a composition which makes use in the most effective way, thus optimizing, the beneficial features of α-lipoic acid in the treatment of neuropathies by reducing as much as possible the total quantity of active substances having an antioxidating effect, thus preferably avoiding the need of many daily administrations.

It is therefore an object of the present invention to provide a composition particularly useful in the diet of subjects suffering from poly-neuropathies, which allows to meet said need, and/or to allow further advantages.

Said object is achieved according to the present invention by a composition whose main features are disclosed in the first claim, while other features are disclosed in the claims 2 to 7.

Said object is also achieved by means of a kit for the treatment of poly-neuropathies comprising α-lipoic acid and SOD, and by means of a food supplement in the form of said kit.

More particularly, the main features of said kit are specified in claim 8 and other features of the kit arc specified in claims 9 to 14. Claim 15 relates to the said composition for a specific therapeutic indication. Claim 16 relates to a food supplement containing said composition in addition to a suitable carrier for alimentation. Claims 17 to 23 relate to further features of said food supplement in kit form.

The composition according to the present invention comprises two substances which are per se already known but they are associated in a new and balanced way suitably for administration to subjects suffering from ncuropathics. In other words, the composition according to the present invention offers the advantage the properties of α-lipoic acid are exploited at best in the treatment of neuropathies, and allows, if necessary, a satisfying decrease of the substances having an anti-oxidant effect, and therefore also a notable reduction of the daily dosed to be administered.

In particular, the composition according to the present invention offers the advantage of an increase of the efficacy of the components α-lipoic acid and SOD, due to an unexpected complementary and synergistic effect.

Another advantage of the composition according to the present invention is that the two components can be administered together, thus obtaining clear advantages from the point of view of administration case, lower number of dosage units to be taken, and minimum hindrance of the dose.

Preferably, the synergic effect is allowed by a substantially simultaneous or concomitant, or alternatively consecutive, ingestion of the two components. For this purpose of simultaneous ingestion, the two components are associated together in the same dosage unit, in the form of a tablet, capsule, micropellets, or similar dosage units.

It is to be noted that said ratio is lower and has a the lower limit of than that of the composition known from US2003/0228299 for the treatment and /or prevention of diseases of the eye surface. This datum is a clear evidence that the applicant has found the existence of said synergic effect, due to the substantially simultaneous ingestion of α-lipoic acid and SOD with respect to the separated and independent use of the single substances, without a specific correlation. In other words, thanks to the synergic effect it is possible to use notably reduced quantities of the two substances, with respect to the quantities known from the above mentioned known document. Said synergic effect has been verified through a number of experimental tests of which three examples will be given in the following.

The first component for the food supplement according to the invention is α-lipoic acid, a natural substance whose antioxidating properties are already known and which therefore does not need a detailed description. In a preferred embodiment α-lipoic acid is present in the composition in a quantity included between 300 mg and 1200 mg, and preferably between 300 mg and 600 mg, according to the dose of assumption and of the form of administration.

The other component of the food supplement according to the present invention is the SOD enzyme, also a natural substance whose properties are already known and therefore do not require a specific detailed description. In a preferred embodiment of the present invention, SOD is introduced in the food supplement in quantities included between 0,2 mg and 10 mg, preferably between 1 mg and 5 mg, according to the dose of assumption and of the form of administration.

The SOD enzyme is a substance of natural origin that can be industrially obtained by extraction of the juice of ripe melon cultivar, or by extraction and purification of biomass derived from seaweed spirulina. The product derived from the extraction of melon juice is a mixture of enzymes, or enzymatic pool, having an antioxidant effect with prevalence of SOD. The product dcriving from biomass is SOD having high grade of purity. For this purpose, it was preferred to use the enzymatic pool for the high antioxidant capacity and for the higher stability of the product which is available on the market in microencapsulated form.

In a preferred embodiment of the invention, the two components α-lipoic acid and SOD are the only active principles, for example the only antioxidants, of the composition. This embodiment has the advantage of allowing a further reduction of the weight and volume of the dosage unit of the food supplement.

In another embodiment of the present invention, the supplement may comprise a third component having the purpose of advantageously modifying some of the therapeutic features thereof. For example, as a third component lactoferrin can be used, a natural substance extracted from human or bovine milk. It is already known that this substance has the property to chelate two ferric ions per molecule, thus preventing the formation of oxygen toxic species. Therefore, it does not require a specific detailed description.

In a preferred embodiment of the invention, lactoferrin is contained in the food supplement in quantities included between 1 and 50 mg, preferably between 30 and 40 mg, according to the dose of assumption and of the form of administration.

The composition according to the present invention can be in form of a kit comprising α-lipoic acid and SOD for a concomitant or consecutive use, such as for instance bags, tablets, micropellets, in liquid or solid form or any other form suitable for treatment of neuropathies, and in particular poly-neuropathies ascribable to degenerative processes caused by the formation of free radicals.

The composition according to the present invention may also be used in addition to a suitable carrier for alimentation in order to form a food supplement, particularly a food supplement intended to complete the diet of subjects suffering from neuropathies.

As above mentioned, in order to prove the synergy of the two components of the composition, the following shows the results of tests carried out on samples of α-lipoic acid and SOD singularly treated, treated in combination in three samples at different dosages, and with addition of lactoferrin, as third specific component.

### EXAMPLE 1

The test was carried out in vitro on a cell line of dopaminergic neurons, named SH-SY5Y, which in vivo represent the target of peripheral neuropathies. The above mentioned cells were cultivated and stimulated with external agents to the purpose of producing reactive species of oxygen and nitrogen so as to reproduce conditions similar to those described in vivo in neuropathies. Then, two parameters were analyzed for evaluating the neuroprotective effects of the tested substances, that is apoptotic neuronal death and total antioxidating activity (TAA), as index of the cellular antioxidating state (ABTS test).

The cells were cultivated in a DMEM growth medium (Dulbecco's modified Eagle's Medium) containing 10% of FBS (Fetal Bovine Serum) previously deactivated at 57°C for 30 minutes, 1% of Penicillin-Streptomycin (Penicillin 5000 UI and Streptomycin 5 mg/ml) and 1% of L-Glutamine (previously prepared by dissolving 0,146 g of glutamine in 5 ml of bidistilled H₂O and by filtering with a filter 0,22 µm of cellulose acetate) in plates for cellular growth having a diameter of 100 mm and treated according to the following procedure:
1) cell growth in the above said growth medium based on DMEM in humidified atmosphere at 37°C with 5% CO₂;
2) detachment of the cells when arrived at the so-called state of detachment with a solution of EDTA-Trypsin 0,02-0,03% and dilution 1:5 or 1:10 in new plates having the same diameter;
3) cell removal and counting with a Burkcr chamber, after coloring with Trypan blue.

The study of apoptosis was carried out in plates containing 12 ml of a cell suspension of the cells at the density of 1,25·10⁵ cells/ml.

The study of the antioxidating activity was carried out with further new plates containing 12 ml of a cell suspension of the cells at the density of 3,5·10⁵ cells/ml.

The plates prepared as above described were additioned with α-lipoic acid or SOD in liquid solution in the relevant concentration to be studied.

The plates were subjected to further incubation for 24 hours at 37°C and 5% CO₂ in order to allow formation of a cell mono-layer.

The cell culture step is followed by stimulation in order to promote oxidative stress by using a neurotoxin, 6-hydroxydopamine (6-OHDA), at the concentration of 100 µM or hydrogen peroxide H₂O₂ at the concentration of 300 µM.

Then, analysis of the cell cultures followed. Neuronal death of apoptotic type was analyzed by the TUNEL method which uses the immunofluorescence technique in order to determine the number of apoptotic nuclei characterized by DNA fragmentation. In detail, the Insitu Cell death Detection Kit, TMR (Rochc Diagnostics, Mannhcim, Germany) was used, which shows the fragmented DNA through a fluorophore which absorbs in the red region, which was then analyzed by fluorescence microscopy. The apoptosis degree was determined as ratio between the number of the apoptosis TUNEL positive nuclei and the total number of the nuclei.

The TAA was evaluated by using an extinction radical, the radical cation of 2,2'-amino-bis(3-ethylbenzothiazoline-6-sulfonic) acid (ABTS) a blue/green chromophore having characteristic absorption at 734 nm.

In the studies, a product provided by Labochim S.p.A. Milano was used as α-lipoic acid (racemic product R, S) and a product provided by the French company BIONOV named EXTRAMEL, available in microencapsulated granules and having a SOD guaranteed activity of 14000 Ul/g, was used as SOD. The growth medium was prepared based on DMEM by BioWhittaker, Walkersville, MD, USA, with additions of FBS by Sigma Chemical, St. Luis, MO, USA and glutamine of the same supplier.

Samples of three concentrations were analyzed both for α-lipoic acid, and for SOD, for the purpose of finding for each component the minimum concentration effective in reducing the production of reactive oxygen species and apoptosis and free from cytotoxic effects. Tables 1 and 2 give the results of these experiments for α-lipoic acid and SOD respectively.

**Table 1**

| α-lipoic acid single dosage | 3mg/ml | 6mg/ml | 12mg/ml |
|---|---|---|---|
| Apoptosis reduction | -12% | -25% | -28% |
| Frcc radicals total reduction | -15% | -27% | -28% |

**Table 2**

| SOD single dosage | 2µg/ml | 20µg/ml | 100µg/ml |
|---|---|---|---|
| Apoptosis reduction | -8% | -15% | -25% |
| Free radicals total reduction | -11% | -29% | -32% |

### EXAMPLE 2

The same substances, active principles and human neuronal cells prepared and stimulated as described in example I were used. In three different samples, quantities of α-lipoic acid and SOD were used, for each sample corresponding respectively to dosages of 300 mg and 0,2 mg, 600 mg and 2 mg, 1200 mg and 10 mg of one dosage unit of the food supplement. The used concentrations were lower- than the values which are considered to be cytotoxic for the two components, and are indicated in table 3.

The association of α-lipoic acid and SOD for each sample showed in all the tested concentrations a surprising synergic effect with respect to the substances used alone in the same dosages. The synergic effect can be summarized in the following Table 3, which shows the percentages of reduction of apoptosis and of the free radicals:

**Table 3**

| Combined dosage | sample 1 | samples 2 | sample 3 |
|---|---|---|---|
| α-lipoic acid dosage | 3 mg/ml | 6 mg/ml | 12 mg/ml |
| SOD dosage | 2 µg/ml | 20 µg/ml | 100µg/ml |
| Apoptosis reduction | -30% | -67% | -76% |
| Free radical total reduction | -41% | -69% | -78% |

The apoptosis, evaluated as reduction of the TUNEL positive apoptotic nuclei number, was significantly lower than the results of example 1. The results show an improvement of nearly three times in terms of free radical total reduction with respect to the results of example 1.

### EXAMPLE 3

The same substances, active principles and human neuronal cells prepared and stimulated as in example 1 were used. In three different samples, quantities of α-lipoic acid, SOD and lactoferrin were used, for each sample corresponding respectively to final dosages of a dosage unit of 300 mg, 0,2 mg, and 1 mg; 600 mg, 2 mg, and 10 mg; 1200 mg, 10 mg and 50 mg. The used concentrations were selected below the values which are considered to be citotoxic for the two components, and are indicated in table 4. A bovine lactoferrin supplied by Morinaga Milk Industries, Japan was used.

From the results shown in Table 4 it is apparent that the addition of lactoferrin to the composition of α-lipoic acid and SOD adds further advantages.

**Table 4**

| Combined dosage | sample 1 | sample 2 | sample 3 |
|---|---|---|---|
| α-lipoic acid dosage | 3 mg/ml | 6 mg/ml | 12 mg/ml |
| SOD dosage | 2 µg/ml | 20 µg/ml | 100 µg/ml |
| Lactoferrin dosage | 10 µg/ml | 100 µg/ml | 500 µg/ml |
| Apoptosis reduction | -35% | -70% | -81% |
| Free radicals total reduction | -45% | -73% | -86% |

The quantitative determination of the oxygen reactive species and of the apoptosis were carried out according to analytical international procedures briefly described in example 1.

Thanks to the synergic effect of α-lipoic acid and SOD, preferably obtained through a single daily dosage unit, the food supplement according to the present invention has proven to be efficacious in the treatment of neuropathies at remarkably lower concentrations than those of the known art, which makes possible the administration to the patient in a single daily dose.

Depending on the dose and the administration form, the food supplement according to the present invention can also comprise other substances having antioxidant effect such as for example Vitamin E and Vitamin C or, as above mentioned, human or bovine lactoferrin and excipients, flavors and other substances having known activity in function of the desired object. Said substances are for example maltodextrin, microcrystalline cellulose, magnesium stearate, colloidal silica, hydroxypropyl methylcellulose, polyvynilpyrrolidone and its derivatives, sodium crosscaramellose, etc. The optimal quantities of said substances also have to be selected on a case by case basis as a function of the dosage and of the administration form of the composition according to the present invention.

The preparation technique of the composition according to the present invention is also selected as a function of the administration type and other practical considerations as shown in the following examples.

### EXAMPLE 4

### Rapid release tablet

For the preparation of a food supplement in a dosage unit in the form of rapid release deglutible tablets containing 600 mg of α-lipoic acid and 20 mg of microencapsulated SOD which is equivalent to 4 mg of enzyme, 5 mg of Vitamin E, 10 mg of lactoferrin, the following steps were carried out:
a) fluid bed granulating α-lipoic acid , maltodextrin and polyvynilpyrrolidone in a ratio of 90:8:2;
b) mixing the obtained granulated product with di-calcium phosphate, microcrystalline cellulose, microencapsulated SOD (14000 UI/g), Vitamin E CWS 50%, bovine lactoferrin, polyvinylpyrrolidone, crosscaramellose, talc, soya lecithin, magnesium stearate in such a proportion that 1 g of mixture contains 600 mg of α-lipoic acid and 20 mg of microencapsulated SOD 14000 UI/g;
c) compressing in a compressing machine having polytetrafluoroethylene punches in order to improve the detachment of the tablets from the punches. Lozenge punches are preferred since this shape allows easy ingestion;
d) spinning of the tablets in a GS spinning machine with hydroxypropylmethylcellulose, stearic acid, titanium dioxide and dycs.

The obtained product or dosage unit is a tablet having lozenge shape and weight of about 1000 mg, coated and colored in order to improve the aspect and case of assumption.

The obtained tablets have a disintegration time lower than 15 minutes and a very fast dissolution such as to release the α-lipoic acid in less than 30 minutes, whereas the microencapsulated SOD with fat substances may pass the gastric barrier.

### EXAMPLE 5

### Bag preparation

In order to prepare the product in monodoses containing 1200 mg of α-lipoic acid and 50 mg of granular SOD which is equivalent to 10 mg of enzyme, the following procedure may be followed.

With suitable spheronizer, a mixture of α-lipoic acid, maltodextrins, polyvinylpyrrolidone, and carboxymethylcellulose is wet spheronized in a preferential ratio of 70: 28,5:1:0,5 in order to obtain a thin granulate with particle size between 300 µm and 1 mm. The granulate obtained by the spheronizer is dried with hot air in a Glatt type fluid bed granulator until a residual humidity lower than 0,5% is obtained.

The so obtained granulate is mixed in suitable proportions in a biconical or V-mixer with maltodextrin, sugar, carboxymethylcellulose, favors, granular microencapsulated SOD. One kg of the final mixture has the following composition: granular α-lipoic acid 38,08%; sugar 43,32%; maltodextrin 10,82%; carboxymethylcellulose 4,44%, Flavor 2,22%, microencapsulated SOD 1,2%.

The thus obtained homogeneous mixture is divided into monodose bags of monocoupled of paper-aluminum-polyethylene or other suitable material by means of a Marchesini RC600 filling machine or other filling machine for pharmaceutical use. The division if carried out by dosing 4,5 g of mixture per single bag so that the average content in α-lipoic acid and microencapsulated SOD is of about 1200 mg and 50 mg, respectively.

The content of one bag may be suspended in about 150 ml of water for preparing a beverage of easy oral ingestion.

### EXAMPLE 6

### Controlled release micropellets

In order to obtain a controlled release of the active principles, preparation of micropellets may be carried out, as dosage units containing the active principles and coated with substances allowed by the food regulations which allow a slow and controlled release.

This technology is very well known for pharmaceutical preparations and consists in depositing on sugar or other substance micropellets the active principles that are then also coated with excipients such as shellac and other excipients for alimentary use such as magnesium stearate, talc, microcrystalline cellulose, etc, which, being gastro resistant, delays the active principles release in the first intestine.

Micropellets containing active principles and excipients in a ratio of 30:70 may be prepared.

Mixtures of micropellets with a different shellac coating may have different release profiles.

For example, three grams of micropellets may contain 900 mg of α-lipoic acid

Microencapsulated SOD in granular form is mixed in a V-mixer in order to obtain a mixture f SOD/micropellets having variable proportions.

For example, 30% α-lipoic acid micropellets and granular microencapsulated SOD may be mixed in the proportion of 98,35% and 1,65%.

The micropellets may be used for filling gel capsule in the following proportions:
Type "OO" capsule: 610 mg of mixture containing about 180 mg of α-lipoic acid and 10 mg of SOD.
Type "O" capsule: 340 mg of mixture containing about 100 mg of α-lipoic acid and 5,6 mg of SOD.

In order to reach higher dosages, the micropellets may be dispersed in a mixture of sorbitol and carrageenans with flavors and intensive sweeteners for preparing an extemporaneous beverage which is thickened by the carrageenans so that the micropellets remain in suspension and can be easily assumed.

For example, 2 g of micropellets and 20 g of granular SOD which is equivalent to 4 mg of enzyme may be mixed with 6 g of a mixture containing sorbitol and thickening agents. Said preparation, suitably packaged in a single-dose bag, may be suspended in 100 ml of water and is suitable for administration of 600 mg of α-lipoic acid and 20 g of granular SOD.

## Claims

1. Composition for use in a method for treatment of poly-neuropathies, **characterized by** comprising α-lipoic acid and SOD (SUPEROXIDE DISMUTASE).

2. Composition for use according to claim 1, in which α-lipoic acid and SOD are contained in the same dosage unit.

3. Composition for use according to claim 1 or 2, in which α-lipoic acid and SOD are the only active ingredients.

4. Composition for use according to any of the preceding claims, in which α-lipoic acid is comprised in amounts between 300 mg and 1200 mg and SOD enzyme is comprised in amounts between 0,2 mg and 10 mg.

5. Composition for use according to claim 4, in which α-lipoic acid is comprised in amounts between 300 mg and 600 mg and SOD is comprised in amounts between 1 mg and 5 mg.

6. Composition for use according to the claims 1, 2, 4 or 6, in which the composition also contains lactoferrin.

7. Composition for use according to claim 6, in which lactoferrin is comprised in amounts between 1 and 50 mg.

8. Kit for use in a method for treatment of poly-neuropathies comprising α-lipoic acid and SOD for simultaneous or consecutive use.

9. Kit for use according to claim 8, in which α-lipoic acid and SOD are contained in the same dosage unit.

10. Kit for use according to claims 8 or 9, in which α-lipoic acid and SOD are the only active ingredients.

11. Kit for use according to any of the preceding claims from 8 to 10, in which α-lipoic acid is comprised in amounts between 300 mg and 1200 mg and SOD is comprised in amounts between 0,2 mg and 10 mg.

12. Kit for use according to claim 11, in which α-lipoic acid is comprised in amounts between 300 mg and 600 mg and SOD is comprised in amounts between 1 mg and 5 mg.

13. Kit for use according to the claims 8, 9, 11 or 12, also containing lactoferrin.

14. Kit for use according to claim 13, in which lactoferrin is comprised in amounts between 1 and 50 mg.

15. Composition according to any of the claims from 1 to 7, for use in a method for treatment of neuropathies, and in particular poly-neuropathies, referring to degenerative processes caused by the formation of free radicals.

16. Food supplement for use in a method for treatment of poly- neuropathies containing a composition according to any of the claims from 1 to 7 in addition to a carrier suitable for alimentation.

17. Food supplement for use in a method for treatment of poly- neuropathies in form of a kit according to claim 8.

18. Food supplement for use in a method for treatment of poly- neuropathies in form of a kit according to claim 9.

19. Food supplement for use in a method for treatment of poly- neuropathies in form of a kit according to claim 10.

20. Food supplement for use in a method for treatment of poly- neuropathies in form of a kit according to claim 11.

21. Food supplement for use in a method for treatment of poly- neuropathies in form of a kit according to claim 12.

22. Food supplement for use in a method for treatment of poly- neuropathies in form of a kit according to claim 13.

23. Food supplement for use in a method for treatment of poly- neuropathies in form of a kit according to claim 14.

## Patentansprüche

1. Zusammensetzung für die Anwendung bei einem Verfahren zur Behandlung von Polyneuropathien, **gekennzeichnet durch** α-Liponsäure und SOD (Superociddismutase) als Bestandteile.

2. Zusammensetzung zur Anwendung gemäß Anspruch 1, wobei α-Liponsäure und SOD in der selben Dosiereinheit enthalten sind.

3. Zusammensetzung zur Anwendung gemäß Anspruch 1 oder 2, wobei α-Liponsäure und SOD die einzigen, aktiven Inhaltsstoffe sind.

4. Zusammensetzung zur Anwendung gemäß einem der vorhergehenden Ansprüche, wobei α-Liponsäurein einer Menge zwischen 300 mg und 1200 mg enthalten ist und SOD-Enzym in einer Menge zwischen 0,2 mg und 10 mg enthalten ist.

5. Zusammensetzung zur Anwendung gemäß Anspruch 4, wobei α-Liponsäure in einer Menge zwischen 300 mg und 600 mg enthalten ist und SOD in einer Menge zwischen 1 mg und 5 mg enthalten ist.

6. Zusammensetzung zur Anwendung gemäß den Ansprüchen 1, 2, 4 oder 6, wobei die Zusammensetzung auch Laktoferrin enthält.

7. Zusammensetzung zur Anwendung gemäß Anspruch 6, wobei Laktoferrin in einer Menge zwischen 1 und 50 mg enthalten ist.

8. Set für die Anwendung bei einem Verfahren zur Behandlung von Polyneuropathien, umfassend eine α-Liponsäureund SOD für die gleichzeitige oder aufeinanderfolgende Anwendung.

9. Set zur Anwendung gemäß Anspruch 8, wobei α-Liponsäureund SOD in der selben Dosiereinheit enthalten sind.

10. Set zur Anwendung gemäß Anspruch 8 oder 9, wobei α-Liponsäureund SOD die einzigen, aktiven Inhaltsstoffe sind.

11. Set zur Anwendung gemäß einem der Ansprüche 8 bis 10, wobei α-Liponsäure in einer Menge zwischen 300 mg und 1200 mg enthalten ist und SOD in einer Menge zwischen 0,2 mg und 10 mg enthalten ist.

12. Set zur Anwendung gemäß Anspruch 11, wobei α-Liponsäurein einer Menge zwischen 300 mg und 600 mg enthalten ist und SOD in einer Menge zwischen 1 mg und 5 mg enthalten ist.

13. Set zur Anwendung gemäß den Ansprüchen 8, 9, 11 oder 12, enthaltend auch Laktoferrin.

14. Set zur Anwendung gemäß Anspruch 6, wobei Laktoferrin in einer Menge zwischen 1 und 50 mg enthalten ist.

15. Zusammensetzung zur Anwendung gemäß einem der Ansprüche 1 bis 7, zur Verwendung bei einem Verfahren zur Behandlung von Neuropathien, und insbesondere von Polyneuropathien, mit Bezug auf durch die Bildung freier Radikale veranlasster, degenerativer Prozesse.

16. Nahrungsergänzungsmittel für die Anwendung bei einem Verfahren zur Behandlung von Polyneuropathien, enthaltend eine Zusammensetzung gemäß einem der Ansprüche 1 bis 7 zusätzlich zu einem für die Nahrungsaufnahme geeigneten Träger.

17. Nahrungsergänzungsmittel zur Anwendung bei einem Verfahren zur Behandlung von Polyneuropathien in Form eines Sets gemäß Anspruch 8.

18. Nahrungsergänzungsmittel zur Anwendung bei einem Verfahren zur Behandlung von Polyneuropathien in Form eines Sets gemäß Anspruch 9.

19. Nahrungsergänzungsmittel zur Anwendung bei einem Verfahren zur Behandlung von Polyneuropathien in Form eines Sets gemäß Anspruch 10.

20. Nahrungsergänzungsmittel zur Anwendung bei einem Verfahren zur Behandlung von Polyneuropathien in Form eines Sets gemäß Anspruch 11.

21. Nahrungsergänzungsmittel zur Anwendung bei einem Verfahren zur Behandlung von Polyneuropathien in Form eines Sets gemäß Anspruch 12.

22. Nahrungsergänzungsmittel zur Anwendung bei einem Verfahren zur Behandlung von Polyneuropathien in Form eines Sets gemäß Anspruch 13.

23. Nahrungsergänzungsmittel zur Anwendung bei einem Verfahren zur Behandlung von Polyneuropathien in Form eines Sets gemäß Anspruch 14.

## Revendications

1. Composition destinée à être utilisée dans une méthode de traitement des polyneuropathies, **caractérisée en ce qu'**elle comprend de l'acide α-lipoïque et une SOD (SUPEROXYDE DISMUTASE).

2. Composition destinée à être utilisée selon la revendication 1, dans laquelle l'acide α-lipoïque et la SOD sont contenus dans la même forme pharmaceutique.

3. Composition destinée à être utilisée selon les revendications 1 ou 2, dans laquelle l'acide α-lipoïque et la SOD sont les seuls principes actifs.

4. Composition destinée à être utilisée selon l'une quelconque des revendications précédentes, dans laquelle l'acide α-lipoïque est compris en des quantités entre 300 et 1200 mg et l'enzyme SOD est comprise en des quantités entre 0,2 et 10 mg.

5. Composition destinée à être utilisée selon la revendication 4, dans laquelle l'acide α-lipoïque est compris en des quantités entre 300 et 600 mg et la SOD est comprise en des quantités entre 1 et 5 mg.

6. Composition destinée à être utilisée selon les revendications 1, 2, 4 ou 6, dans laquelle la composition contient également de la lactoferrine.

7. Composition destinée à être utilisée selon la revendication 6, dans laquelle la lactoferrine est comprise en des quantités entre 1 et 50 mg.

8. Kit destiné à être utilisé dans une méthode de traitement des polyneuropathies comprenant de l'acide α-lipoïque et une SOD pour une utilisation simultanée ou consécutive.

9. Kit destiné à être utilisé selon la revendication 8, dans lequel l'acide α-lipoïque et la SOD sont contenus dans la même forme pharmaceutique.

10. Kit destiné à être utilisé selon les revendications 8 ou 9, dans lequel l'acide α-lipoïque et la SOD sont les seuls principes actifs.

11. Kit destiné à être utilisé selon l'une quelconque des revendications 8 à 10, dans lequel l'acide α-lipoïque est compris en des quantités entre 300 et 1200 mg et la SOD est comprise en des quantités entre 0,2 et 10 mg.

12. Kit destiné à être utilisé selon la revendication 11, dans lequel l'acide α-lipoïque est compris en des quantités entre 300 et 600 mg et la SOD est comprise en des quantités entre 1 et 5 mg.

13. Kit destiné à être utilisé selon les revendications 8, 9, 11 ou 12, contenant également de la lactoferrine.

14. Kit destiné à être utilisé selon la revendication 13, dans lequel la lactoferrine est comprise en des quantités entre 1 et 50 mg.

15. Composition selon l'une quelconque des revendications 1 à 7, destinée à être utilisée dans une méthode de traitement des neuropathies, et en particulier des polyneuropathies, en référence aux processus dégénératifs provoqués par la formation de radicaux libres.

16. Complément alimentaire destiné à être utilisé dans une méthode de traitement des polyneuropathies contenant une composition selon l'une quelconque des revendications 1 à 7 en plus d'un véhicule convenable pour l'alimentation.

17. Complément alimentaire destiné à être utilisé dans une méthode de traitement des polyneuropathies sous la forme d'un kit selon la revendication 8.

18. Complément alimentaire destiné à être utilisé dans une méthode de traitement des polyneuropathies sous la forme d'un kit selon la revendication 9.

19. Complément alimentaire destiné à être utilisé dans une méthode de traitement des polyneuropathies sous la forme d'un kit selon la revendication 10.

20. Complément alimentaire destiné à être utilisé dans une méthode de traitement des polyneuropathies sous la forme d'un kit selon la revendication 11.

21. Complément alimentaire destiné à être utilisé dans une méthode de traitement des polyneuropathies sous la forme d'un kit selon la revendication 12.

22. Complément alimentaire destiné à être utilisé dans une méthode de traitement des polyneuropathies sous la forme d'un kit selon la revendication 13.

23. Complément alimentaire destiné à être utilisé dans une méthode de traitement des polyneuropathies sous la forme d'un kit selon la revendication 14.
